# EUROPÄISCHE PATENTSCHRIFT

(11) **EP 1 508 066 B1**
(45) Veröffentlichungstag und Bekanntmachung des Hinweises auf die Patenterteilung: **22.11.2006**
(21) Anmeldenummer: 03732422.5
(22) Anmeldetag: 20.05.2003
(51) Int. Cl.: G02B 23/24, A61B 1/04

(54) **MIKROENDOSKOP**
MICROENDOSCOPE
MICRO-ENDOSCOPE

(30) Priorität: 22.05.2002 DE 10222505
(43) Veröffentlichungstag der Anmeldung: 23.02.2005
(73) Patentinhaber: Schölly Fiberoptic GmbH, 79211 Denzlingen (DE)
(72) Erfinder: SCHÖLLY, Werner, 79211 Denzlingen (DE)
(74) Vertreter: Maucher, Wolfgang
(86) Internationale Anmeldenummer: PCT/EP2003/005274
(87) Internationale Veröffentlichungsnummer: WO 2003/098315

(56) Entgegenhaltungen:
- EP-A- 0 609 093
- WO-A-01/63334
- DE-A1- 4 009 438
- DE-A1- 10 033 142
- DE-A1- 10 155 921
- DE-A1- 19 859 155
- US-A- 4 269 485
- US-A- 4 628 207

## Beschreibung

Die Erfindung bezieht sich auf ein Mikroendoskop mit einer distalen Sondenspitze mit einem Durchmesser von bis zu etwa 1 mm und einem Sondenhandgriff, wobei in der Sondenspitze über die ganze Arbeitslänge eine optische Baugruppe zumindest mit einem Mikroobjektiv und einer anschließenden Mikrolinsen-Baugruppe als Bildübertragungselement untergebracht ist, wobei im Bereich des Sondenhandgriffs an die optische Baugruppe der Sondenspitze eine Bildübertragung anschließt, die einen größeren aktiven Durchmesser als die optische Baugruppe der Sondenspitze hat, wobei zwischen der optischen Baugruppe der Sondenspitze und dem sich anschließenden Bildleiter ein bildvergrößerndes Koppelelement vorgesehen ist.

In der Mikroendoskopie sind bei Arbeitsdurchmessern unter 1 mm flexible Endoskope bekannt, die nahe dem distalen Ende einen leichten Handgriff aufweisen, dessen starre Spitze sich beispielsweise über eine Länge von 30 - 120 mm erstrecken kann. Die Mikroendoskopspitze hat im allgemeinen folgenden Aufbau:
Im Zentrum distal befindet sich ein Mikroobjektiv, bestehend aus einer oder mehreren Mikrolinsen, z. B. mit einem Außendurchmesser von 0,5 mm. Dieses distale Mikroobjektiv ist meist direkt aufgesetzt auf einen nachfolgenden Bildleiter mit gleichem aktivem Durchmesser. Am proximalen Ende wird das vom Bildleiter übertragene pixelstrukturierte Bild von einem Objektiv für Augenbetrachtung oder für die Projektion auf ein VideoKamera-Array bereitgestellt.
Zur Beleuchtung des Bildfeldes sind um das distale Mikroobjektiv und den Bildleiter entlang der Sondenlänge Lichtleitfasern angeordnet, die nahe dem proximalen Ende mechanisch gefasst sind und über einen Verbindungslichtleiter mit einer Lichtquelle verbunden sind. Im Bereich der distalen Spitze sind Mikroobjektiv, Bildleiter und Lichtleitfaser in einem starren oder halbflexiblen Röhrchen eingebaut, das für diese Komponenten Halterung und Führung bildet.
Hinter dem Handgriff zum proximalen Ende hin sind die licht- und bildübertragenden Elemente meist in einem flexiblen Schutzschlauch gefasst. Für bestimmte Anwendungen kann zusätzlich ein Leerkanal, dargestellt zum Beispiel durch einen Mikrolumenschlauch oder ein Metallröhrchen, in die Sonde integriert sein.

Viele mikroendoskopischen Anwendungen in der Medizintechnik und für industrielle Anwendungen erfordern kleine Arbeitsdurchmesser ab 0,15 bis ca. 1 mm. Diese Arbeitsdurchmesser erlauben für den Bildleiter aufgrund der Platzverhältnisse nur ca. 3000 Bildpunkte; die Anwendungen verlangen jedoch höhere Auflösungen mit mehr als mindestens 10 000 Bildpunkten (z.B.20 000, 30 000, 50 000). Außerdem soll der Handgriff der mikroendoskopischen Sonde und die Sondenzuleitung möglichst leicht ausgeführt werden, was die Bildübertragung mit einem flexiblen Bildleitersystem zur Endoskopiekamera (für Video-Betrachtung) erfordert. Bildleiter mit Bildpunktzahlen größer als 3000 ermöglichen keine distalen Sondenspitzen mit Arbeitsdurchmessern von kleiner als 0,5 mm beim oben beschriebenen Aufbau.

Aus der EP-A- 0 609 093 ist eine Betrachtungsanordnung mit drei ineinandergreifenden, rohrförmigen Gehäusen bekannt, in denen ein Objektiv und eine Teilstrecke mit einer Gradientenindex-Linse, Koppellinsen und schließlich ein Bildwandler angeordnet sind. Es werden dabei Gradientenindex-Linsen zur Korrektur der axialen chromatischen Aberration verwendet.

Aus der US-A- 4 628 207 ist ein Endoskop mit einem distalen Objektiv, einer sich anschließenden Bildübertragung mit Stablinsen und schließlich einem Adapter zum Ankoppeln einer Spiegelreflexkamera bekannt. Nachteilig ist hierbei, dass die Kamera über den Adapter starr gekoppelt ist und dadurch eine feinfühlige Manipulation mit dem Endoskop behindert.

Aus der US-A 4 269 485 ist ein optisches Objektivsystem für Endoskope bekannt, bei dem eine Fokussierung durch Verschiebung einer rückseitigen Linsengruppe zusammen mit einem anschließenden Faserbündel gegenüber einer fixen, vorderen Linsengruppe vorgesehen ist.

Aus der WO 0163334 A ist ein integriertes optisches System für Endoskope bekannt. Dieses optische System beinhaltet ein Objektiv und anschließend mehrere Linsenanordnungen und beschreibt diverse Möglichkeiten der Farbkorrektur und der Korrektur der chromatischen Aberration.

Aus der DE 400 9 438 ist ein zahnärztliches Arbeits-Handstück mit einer Bildaufnahme-Einrichtung bekannt. Dabei ist distal eine optische Umlenkung, dann eine Streulichtblende und eine Frontlinse und anschließend ein zu einer Videokamera mit vorgeschaltetem Objektiv führender Bildleiter angeordnet. Eine ausreichende optische Auflösung des Bildleiters kann bei Anwendung in einem zahnärztlichen Handstück, das in seinen Abmessungen, seiner Form und seiner Handhabung den üblichen zahnärztlichen Instrumenten entspricht, durch Einsatz von Bildleitern, die einen größeren Durchmesser als 1mm aufweisen, erreicht werden. Bei sehr dünnen vorderen Enden des Handstückes ist eine hohe Auflösung mit dieser Konstruktion nicht möglich.

Aus der DE 101 55 921 ist eine Bildsuchvorrichtung, die in einer endoskopischen Einrichtung eingebaut ist, bekannt. Diese weist ein starres Endoskop und eine Bildtrennvorrichtung auf, an die eine erste und eine zweite CCD-Kamera angeschlossen sind.

Aus der DE 198 59 155 ist ein Endoskop mit einer Koppeleinrichtung zum Anschluss einer Videokamera oder eines Okulars zur direkten Betrachtung, das nach außen vollständig und dicht abgeschlossen ist, um autoklaviert werden zu können, bekannt. Das Endoskop weist durchgängig ein starres Linsensystem auf, an dessen proximales Ende über eine Koppeleinrichtung starr eine Videokamera oder ein Okular anschließbar sind.

Aus der DE 100 33 142 ist ein Erregerfilter für ein Endoskop zur Fluoreszenzuntersuchung bekannt. Es weist ein optisches Bildaufnahmesystem auf, das ein Fluoreszenzbild einfängt. Dazu wird das Fluoreszenzbild über eine Objektivoptik, ein Lichtwellenleiter-Faserbündel und eine Okularlinse auf ein Bildaufnahmeteil mit einer CCD-Kamera übertragen. Für eine feinmotorische Handhabung ist das Gerät ungeeignet.

Aufgabe der vorliegenden Erfindung ist es, ein Mikroendoskop mit einer Sondenspitze zu schaffen, das bei einem kleinen Sondenspitzen-Durchmesser eine hohe Auflösung ermöglicht und dabei einen leichten Handgriff und Sondenzuleitungen und damit eine feinfühlige, exakte Bewegung der Endoskopsspitze ermöglicht.

Zur Lösung dieser Aufgabe wird vorgeschlagen, dass die Sondenspitze mit der optischen Baugruppe abnehmbar mit dem Sondenhandgriff verbunden ist, und dass die Bildübertragung einen faseroptischen Bildleiter mit hoher Bildpunktezahl von mehr als 10000 Bildpunkten aufweist.

Bei einem solche Mikroendoskop kann die durch die in der dünnen Sondenspitze mit einem Durchmesser von beispielsweise 0,5 mm befindliche optische Baugruppe mit dem Mikroobjektiv und der Mikrolinsen-Baugruppe erzielte, hohe Bildauflösung auf den im Sondenhandgriff befindlichen, faseroptischen Bildleiter übertragen werden, der seinerseits durch seine hohe Bildpunktezahl die hohe Bildauflösung der Sondenspitze nicht beeinträchtigt. Über den faseroptischen Bildleiter ist eine Bildübertragung auf einen CCD-Bildwandler einer externen Endoskopie-Kamera möglich. Dadurch ist die Masse des Mikroendoskops selbst gering, was eine feinfühlige, fingergesteuerte, exakte Bewegbarkeit der Sondenspitze begünstigt.
Durch die Abnehmbarkeit der miniaturisierten Sondenspitze vom Handgriff kann sie für medizintechnische Anwendungen auch dampfsterilisierbar ausgeführt sein.
Typische Handgriffe für mikroendoskopische Sonden können Außendurchmesser von ca. 8 - 10 mm auf einer Länge von >50 mm haben.

Nach einer Ausgestaltung der Erfindung kann in das Mikroendoskop ein Leerkanal für Spülung und/oder Applikatoren wie Laserfasern integriert sein.

Nachstehend ist die Erfindung mit ihren wesentlichen Einzelheiten anhand der Zeichnung noch näher erläutert.

Es zeigt:

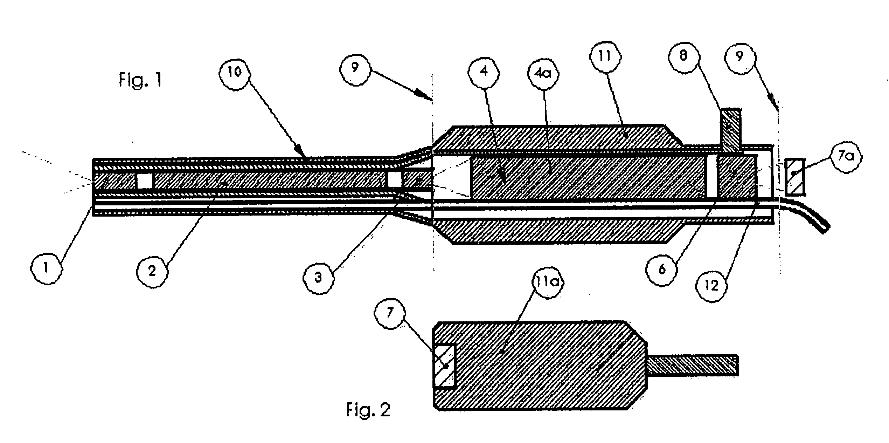

- Fig.1: eine Längsschnittansicht eines Mikroendoskops.

Der erfindungsgemäße Aufbau eines Mikroendoskops gemäß Fig.1 ist wie folgt:
Das distale Objektiv 1, das aus einer oder mehreren mikrooptischen Komponenten, zum Beispiel einer Gradientenindexlinse bestehen kann, ist einem bildübertragenden Stablinsensystem 2 aus einem oder mehreren Elementen, vorzugsweise einem Gradientenstab, vorgeschaltet. Das optische Koppelelement 3 bildet das von dem distalen Objektiv 1 und dem Stablinsensystem 2 übertragene Bild formatfüllend auf den wesentlich größeren aktiven Bildfelddurchmesser des Bildleiters 4a als Bildübertragung 4 ab. Proximal ist ein weiteres Objektiv 6 vorgesehen, das auf die Bildfeldgröße des CCD-Bildwandlers 7a einer Endoskopie-Kamera etwa formatfüllend abbildet.
Im Sondenspitzenbereich sind konzentrisch Lichtleitfasern zur Beleuchtung des Bildfeldes angeordnet, die an einer Trennstelle 9 vorzugsweise als Ringlicht oder als Ein- oder Mehrpunktübergabe realisiert sein können. Mit 8 ist das hintere Lichtleiterende bezeichnet.
In der Ausführung mit Trennstelle kann das Koppelelement 3 entweder in der Sondenspitze 10 oder im Handgriffteil 11 integriert sein. Die abnehmbaren Sondenspitzen können mit unterschiedlichen Bildwinkeln und Blickrichtungen realisiert werden.

## Patentansprüche

1. Mikroendoskop mit einer distalen Sondenspitze (10), die einen Durchmesser von bis zu etwa 1mm aufweist, und einem Sondenhandgriff (11), wobei in der Sondenspitze (10) über die ganze Arbeitslänge eine optische Baugruppe zumindest mit einem Mikroobjektiv (1) und einer anschließenden Mikrolinsen-Baugruppe (2) als Bildübertragungselement untergebracht ist, wobei im Bereich des Sondenhandgriffs (11) an die optische Baugruppe der Sondenspitze (10) eine Bildübertragung (4) anschließt, die einen größeren aktiven Durchmesser als die optische Baugruppe der Sondenspitze (10) hat, wobei zwischen der optischen Baugruppe der Sondenspitze und dem sich anschließenden Bildleiter (4a) ein bildvergrößerndes Koppelelement (3) vorgesehen ist, **dadurch gekennzeichnet, dass** die Sondenspitze mit der optischen Baugruppe abnehmbar mit dem Sondenhandgriff (11) verbunden ist, und dass die Bildübertragung (4) einen faseroptischen Bildleiter (4a) mit hoher Bildpunktezahl von mehr als 10000 Bildpunkten aufweist.

2. Mikroendoskop nach Anspruch 1, **dadurch gekennzeichnet, dass** in das Mikroendoskop ein Leerkanal (12) für Spülung und/oder Applikatoren wie Laserfasern integriert ist.

## Claims

1. Microendoscope having a distal probe tip (10), which has a diameter of up to about 1 mm, and a probe handle (11), wherein an optical assembly at least comprising a microobjective (1) and an adjacent microlens assembly (2) as an image transmitting element is accommodated in the probe tip (10) over the entire operating length, while in the region of the probe handle (11) adjoining the optical assembly of the probe tip (10) is an image transmitting device (4) which has a greater active diameter than the optical assembly of the probe tip (10), while between the optical assembly of the probe tip and the adjoining image conductor (4a) is provided an image enlarging coupling element (3), **characterised in that** the probe tip with the optical assembly is removably connected to the probe handle (11), and **in that** the image transmitting device (4) has a fibre-optical image conductor (4a) with a high pixel number of more than 10000 pixels.

2. Microendoscope according to claim 1, **characterised in that** an empty channel (12) for irrigation purposes and/or for applicators such as laser fibres is integrated in the microendoscope.

## Revendications

1. Micro-endoscope comprenant une pointe de sonde (10) qui présente un diamètre pouvant atteindre environ 1 mm et une poignée de sonde (11), dans lequel un bloc optique comprenant au moins un micro-objectif (1) et un bloc de microlentilles (2) lui faisant suite est logé en tant qu'élément de transmission d'image dans la pointe de sonde (10) sur toute la longueur de travail, dans lequel un dispositif de transmission d'image (4), qui a un plus grand diamètre actif que le bloc optique de la pointe de sonde (10), fait suite au bloc optique de la pointe de sonde (10) au niveau de la poignée de sonde (11), dans lequel un élément de couplage agrandisseur d'image est prévu entre le bloc optique de la pointe de sonde et le guide d'image (4a) qui lui fait suite, **caractérisé par le fait que** la pointe de sonde avec le bloc optique est assemblée de manière amovible avec la poignée de sonde (11) et que le dispositif de transmission d'image (4) présente un guide d'image (4a) à fibres optiques avec un nombre de points d'image supérieur à 10 000 points d'image.

2. Micro-endoscope selon la revendication 1, **caractérisé par le fait qu'**un canal vide (12) pour le rinçage et/ou des applicateurs tels que fibres laser est intégré dans le micro-endoscope.
